(19) Europäisches Patentamt
European Patent Office
Office européen des brevets

(11) **EP 4 268 942 A1**

(12) **EUROPEAN PATENT APPLICATION**
published in accordance with Art. 153(4) EPC

(43) Date of publication:
**01.11.2023 Bulletin 2023/44**

(21) Application number: **21910311.6**

(22) Date of filing: **08.12.2021**

(51) International Patent Classification (IPC):
*B01D 59/04* (2006.01)    *C01B 21/24* (2006.01)
*C07C 17/383* (2006.01)    *C07C 19/08* (2006.01)

(52) Cooperative Patent Classification (CPC):
**B01D 59/04; C01B 21/24; C07C 17/383;
C07C 19/08**

(86) International application number:
**PCT/JP2021/045097**

(87) International publication number:
**WO 2022/138162 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.12.2020 JP 2020214563**

(71) Applicant: **Taiyo Nippon Sanso Corporation
Tokyo 142-8558 (JP)**

(72) Inventors:
• **SAKURAI, Hayato
Tokyo 142-8558 (JP)**
• **KAMBE, Takashi
Tokyo 142-8558 (JP)**

(74) Representative: **HGF
HGF Limited
1 City Walk
Leeds LS11 9DX (GB)**

(54) **STABLE ISOTOPE ENRICHMENT DEVICE AND STABLE ISOTOPE ENRICHMENT METHOD**

(57)    The object of the present invention is to provide a stable isotope enrichment device and a stable isotope enrichment method capable of reducing the discharge amount of toxic or combustible substances or substances causing environmental load in the atmosphere and reducing the amount of raw materials used. The present invention provides a stable isotope enrichment device, including: a distillation column group in which a plurality of distillation columns are connected in a cascade; a raw material supply line (30) that supplies a raw material into a first distillation column (1); a product line (31) that withdraws a product from another distillation column located on the secondary side of the first distillation column (1); an isotope-depleted fluid withdraw line (32) that withdraws an isotope-depleted gas or an isotope-depleted liquid from the first distillation column (1) or another distillation column located on the primary side of the one distillation column (1); an isotope exchange reactor (22) at which the isotope-depleted gas or the isotope-depleted liquid is subjected to an isotope exchange reaction, the isotope-depleted gas or the isotope-depleted liquid is regenerated such that the concentration of molecules containing a target stable isotope in the isotope-depleted gas or the isotope-depleted liquid approaches the natural abundance ratio, and an isotope-regenerated gas or an isotope-regenerated liquid is produced; and an isotope-regenerated fluid return line (33) that re-supplies the isotope-regenerated gas or the isotope-regenerated liquid into the first distillation column (1).

FIG. 1

**Description**

TECHNICAL FIELD

The present invention relates to a stable isotope enrichment device and a stable isotope enrichment method which enriches a stable isotope.

BACKGROUND ART

Stable isotopes are used in the fields of organic chemistry and biochemistry to obtain information on various reactions, structures and functions of organic substances and biological substances. Specifically, stable isotopes are used as a tracer in NMR analysis in the fields of natural science and medicine, in drug discovery and organic synthesis using the kinetic isotope effect in which the reaction rate changes due to isotope substitution, in infrared and Raman spectroscopy using the difference in optical properties, and the like.

There are various methods for separating stable isotopes, such as thermal diffusion separation, centrifugation, laser separation, chemical exchange separation, and distillation separation. Among these methods, the distillation separation method is suitable for mass production of isotopes of light elements. For this reason, for example, methods such as distillation separation of water and distillation separation of oxygen are employed as industrial methods for separating stable isotopes of oxygen.

For example, Patent Document 1 discloses a method and a device for producing heavy oxygen water by combining oxygen distillation and water distillation. As disclosed in Patent Document 1, oxygen gas and water vapor in which the concentration of the components containing target heavy isotope (oxygen molecule containing $^{17}O$ or $^{18}O$) has been decreased is discharged from the top of the distillation column to the outside of the system. Since these components are all harmless, the oxygen gas is diffused into the atmosphere and the water vapor is treated as discharged water.

Note that, a stable isotope used as a tracer and a stable isotope used in organic synthesis and drug discovery are required to have a high concentration of 95 to 99% or more. Since the natural abundance ratio of $^{18}O$ is about 2000 ppm, a large amount of discharged gas and discharged liquid is generated when high enrichment is performed by the distillation separation method described in Patent Document 1. However, as described in Patent Document 1, if the discharged gas and the discharged liquid are harmless oxygen or water, there is no problem in terms of environmental load.

Further, as a method for enriching carbon stable isotope, there is a method of separating carbon monoxide, methane, carbon tetrafluoride, and the like by distillation. For example, Patent Document 2 discloses a carbon monoxide stable isotope enrichment device and enrichment method for enriching $^{13}CO$.

Even when the carbon stable isotope is enriched using the technology disclosed in Patent Document 2, a large amount of CO gas of which the concentration of $^{13}CO$ is lower than the natural abundance ratio is discharged outside the system from the top of the first distillation column, as in the case of Patent Document 1 described above (hereinafter, the discharged gas or discharged liquid of which the concentration of molecules containing a target stable isotope is lower than the natural abundance ratio and is discharged from the stable isotope enrichment device may be referred to as "isotope-depleted gas" or "isotope-depleted liquid".). In this case, since carbon monoxide is combustible and toxic, it cannot be discharged into the atmosphere as it is, and it is necessary to carry out a detoxification treatment.

Examples of an industrial detoxification treatment include a method using a flare stack and a method using an oxidation treatment by a catalytic reaction.

For example, Patent Document 3 discloses a carbon monoxide removal unit and carbon monoxide removal method for converting carbon monoxide into carbon dioxide by a catalytic reaction as the method using oxidation treatment by a catalytic reaction.

All of the methods above convert carbon monoxide into carbon dioxide by oxidizing. As a result, a large amount of carbon dioxide will be discharged into the atmosphere, resulting in an environmental load.

Also, in the removal treatments above, even when methane is separated by distillation to enrich carbon stable isotopes, a large amount of carbon dioxide is discharged into the atmosphere in the same manner as described above.

Moreover, carbon tetrafluoride is not combustible or toxic, so there is no need to remove it. However, since the global warming potential of carbon tetrafluoride is as high as about 7400, there is also the problem that the environment load is greater. The global warming potential is one of many indicators of environmental load.

In addition, it is conceivable that other stable isotopes will have the same problems as described below.

For example, nitrogen monoxide distillation is a method for enriching nitrogen stable isotopes. This method is a highly convenient method in which nitrogen isotopes and oxygen isotopes can be obtained at the same time. As described in Non-Patent Document 1, it is known that there were plants in the United States that co-produce high concentrations of heavy oxygen isotopes of 95% or more and heavy nitrogen isotopes. However, nitric oxide is thought to cause acid rain and photochemical smog as nitrogen oxides, and is toxic. For this reason, as with carbon monoxide, it cannot be directly discharged into the atmosphere, so a removal treatment such as an ammonia catalytic reduction method is required.

PRIOR ART DOCUMENTS

PATENT DOCUMENTS

Patent Document 1: Japanese Patent No. 4327287
Patent document 2: Japanese Unexamined Patent Application, First Publication No. 2018-164884
Patent Document 3: Japanese Patent No. 4309162

NON-PATENT DOCUMENTS

Non-Patent Document 1: Shohei Isomura, Yasuhiko Tonooka, Hayato Kaetsu, "Separation of Nitrogen and Oxygen Isotopes by Nitric Oxide Low Temperature Distillation (RADIO ISOTOPES: Vol. 36, No. 2)", Japan Radioisotope Association, February 15, 1987, P57 -63

SUMMARY OF INVENTION

PROBLEM TO BE SOLVED BY THE INVENTION

The present invention has been made in view of the above problems, and the object of the present invention is to provide a stable isotope enrichment device and a stable isotope enrichment method capable of reducing the discharge amount of toxic or combustible substances or substances causing environmental load in the atmosphere and reducing the amount of raw materials used.

MEANS FOR SOLVING THE PROBLEM

The inventors of the present invention have made extensive studies to solve the problems above. As a result, the inventors of the present invention found that when enriching a stable isotope, the isotope-depleted gas or the isotope-depleted liquid in which the concentration of molecules containing the target stable isotope is lower than the natural abundance ratio can be reused as a raw material by increasing the concentration of the molecules containing the target stable isotope in the isotope-depleted gas or the isotope-depleted liquid approaches close to the natural abundance ratio by an isotope exchange reaction. As a result, the inventors have found that it is possible to decrease the amount of the raw material used while reducing the discharge amount of substances that cause environmental load into the atmosphere, and have completed the present invention.

That is, the present invention provides the following stable isotope enrichment devices and stable isotope enrichment methods.

(1) A stable isotope enrichment device for enriching a stable isotope by distillation, including:

a distillation column group in which a plurality of distillation columns are connected in a cascade;
a raw material supply line that supplies a raw material into one of the distillation columns in the distillation column group;
a product line that withdraws a product from another distillation column located on the secondary side of the one distillation column in the distillation column group;
an isotope-depleted fluid withdraw line that withdraws an isotope-depleted gas or an isotope-depleted liquid of which a concentration of molecules containing the target stable isotope is lower than a natural abundance ratio, and which is from the one distillation column or another distillation column located on the primary side of the one distillation column in the distillation column group;
an isotope exchange reactor at which the isotope-depleted gas or the isotope-depleted liquid introduced from the isotope-depleted fluid withdraw line is subjected to an isotope exchange reaction with other substances including the same stable isotope as the target stable isotope, the isotope-depleted gas or the isotope-depleted liquid is regenerated such that the concentration of the molecules containing the target stable isotope in the isotope-depleted gas or the isotope-depleted liquid approaches a natural abundance ratio, and an isotope-regenerated gas or an isotope-regenerated liquid is produced; and
an isotope-regenerated fluid return line that re-supplies the isotope-regenerated gas or the isotope-regenerated liquid produced in the isotope exchange reactor into the one distillation column.

(2) The stable isotope enrichment device according to (1), wherein the isotope exchange reaction in the isotope exchange reactor is any of a catalyst heating reaction, a discharge reaction, an ion exchange method, and a photochemical reaction.

(3) An isotope enrichment method for enriching a stable isotope by distillation, including:
a re-supplying step in which an isotope-depleted gas or an isotope-depleted liquid, of which a concentration of molecules containing the target stable isotope is lower than a natural abundance ratio and which is withdrawn from one distillation column or another distillation column located on the primary side of the one distillation column in a distillation column group in which a plurality of distillation columns are connected in a cascade, is subjected to an isotope exchange reaction with other substances containing the same stable isotope as the target stable isotope, the concentration of molecules containing the target stable isotope in the isotope-depleted gas or the isotope-depleted liquid approaches the natural abundance ratio, the isotope-depleted gas or the isotope-depleted liquid is regenerated, and an isotope-regenerated gas or an isotope-regenerated liquid is produced, and the isotope-regenerated gas or the isotope-regenerated liquid is re-supplied into the one distillation column.

(4) The stable isotope enrichment method according to (3), wherein the isotope exchange reaction in the isotope exchange reactor is any of a catalyst heating reaction, a discharge reaction, an ion exchange method, and a photochemical reaction.

EFFECTS OF THE INVENTION

The stable isotope enrichment device according to the present invention includes the isotope exchange reactor at which the isotope-depleted gas or the isotope-depleted liquid, which is introduced from the one distillation column or another distillation column located on the primary side of the one distillation column and of which the concentration of molecules containing a target stable isotope is lower than the natural abundance ratio, is subjected to the isotope exchange reaction with other substances including the same stable isotope as the target stable isotope, the concentration of the molecules containing the target stable isotope in the isotope-depleted gas or the isotope-depleted liquid approaches the natural abundance ratio, the isotope-depleted gas or the isotope-depleted liquid is regenerated, and an isotope-regenerated gas or an isotope-regenerated liquid is produced.

According to the stable isotope enrichment device of the present invention, since the stable isotope enrichment device of the present invention includes the isotope exchange reactor, it is possible to decrease the discharge amount of toxic or combustible substances or the discharge amount of the isotope-depleted gas or the isotope-depleted liquid including substances causing environmental load in the atmosphere, and reuse these substances as a raw material.

Therefore, it is possible to decrease the amount of the raw material used while reducing the environmental load in the stable isotope enrichment process, so that molecules containing the target stable isotope can be efficiently obtained in a high concentration at low cost while maintaining a favorable process environment.

In addition, the stable isotope enrichment method according to the present invention includes the re-supplying step in which an isotope-depleted gas or an isotope-depleted liquid, of which a concentration of molecules containing a target stable isotope is lower than a natural abundance ratio and which is withdrawn from one distillation column or another distillation column located on the primary side of the one distillation column, is subjected to the isotope exchange reaction with other substances including the same stable isotope as a target stable isotope, the concentration of molecules containing the target stable isotope in the isotope-depleted gas or the isotope-depleted liquid approaches a natural abundance ratio, the isotope-depleted gas or the isotope-depleted liquid is regenerated, and an isotope-regenerated gas or an isotope-regenerated liquid is produced, and the isotope-regenerated gas or the isotope-regenerated liquid is re-supplied into the one distillation column.

According to the stable isotope enrichment method of the present invention, since the stable isotope enrichment method of the present invention includes the re-supplying step in which the isotope exchange reaction is carried out, it is possible to decrease the amount of the raw material used while reducing the environmental load in the stable isotope enrichment process. Therefore, the molecules containing the target stable isotope can be efficiently obtained in a high purity at low cost while maintaining a favorable process environment.

BRIEF DESCRIPTION OF DRAWINGS

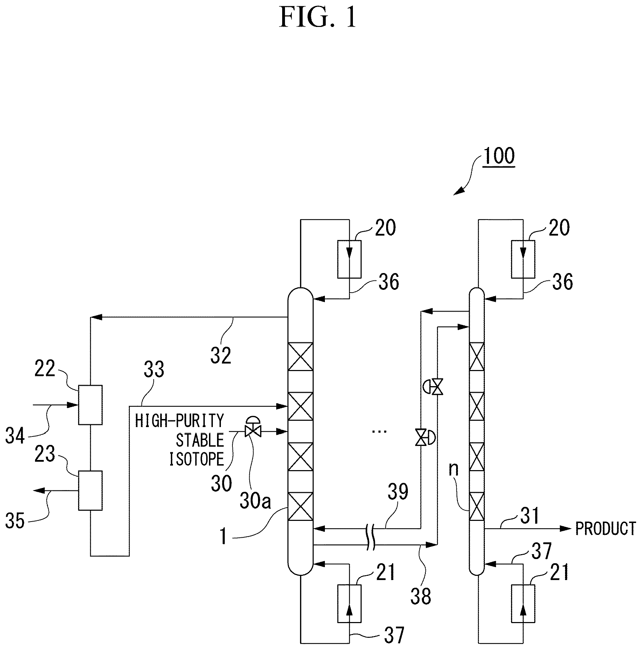

[FIG. 1] FIG. 1 is a diagram schematically illustrating a stable isotope enrichment device and a stable isotope enrichment method according to an embodiment of the present invention, and is a system diagram showing a main portion of the stable isotope enrichment device.

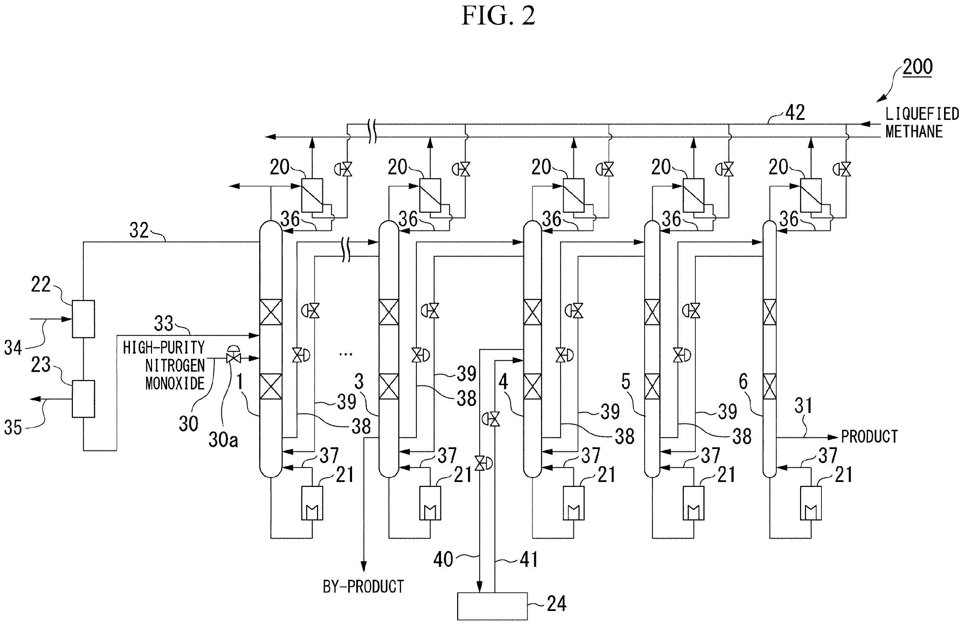

[FIG. 2] FIG. 2 is a diagram schematically illustrating a stable isotope enrichment device used in Example 1 of the present invention, and is a system diagram showing a main portion of the stable isotope enrichment device used for enriching nitrogen monoxide containing a stable isotope in Example 1.

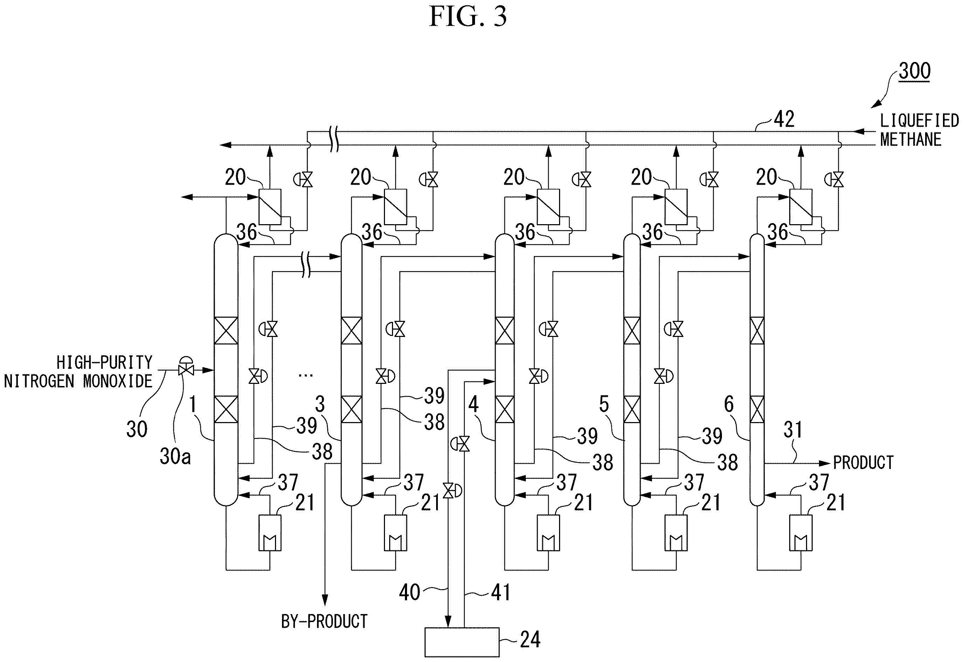

[FIG. 3] FIG. 3 is a diagram schematically illustrating a stable isotope enrichment device used in Comparative Example 1, and is a system diagram showing a main portion of the stable isotope enrichment device used for enriching nitrogen monoxide containing stable isotope in Comparative Example 1.

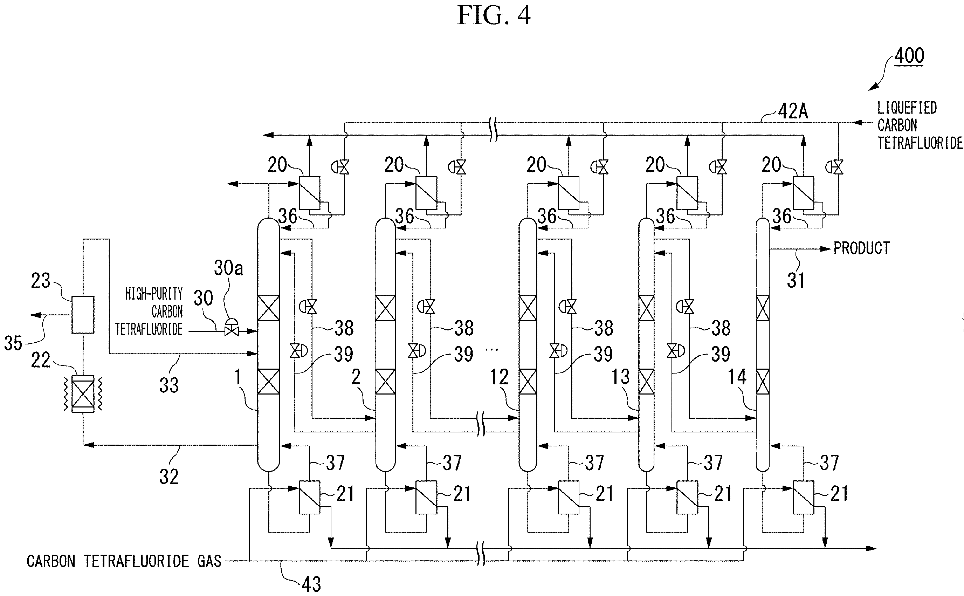

[FIG. 4] FIG. 4 is a diagram schematically illustrating a stable isotope enrichment device used in Example 2 of the present invention, and is a system diagram showing a main portion of the stable isotope enrichment device used for enriching carbon tetrafluoride containing a stable isotope in Example 2.

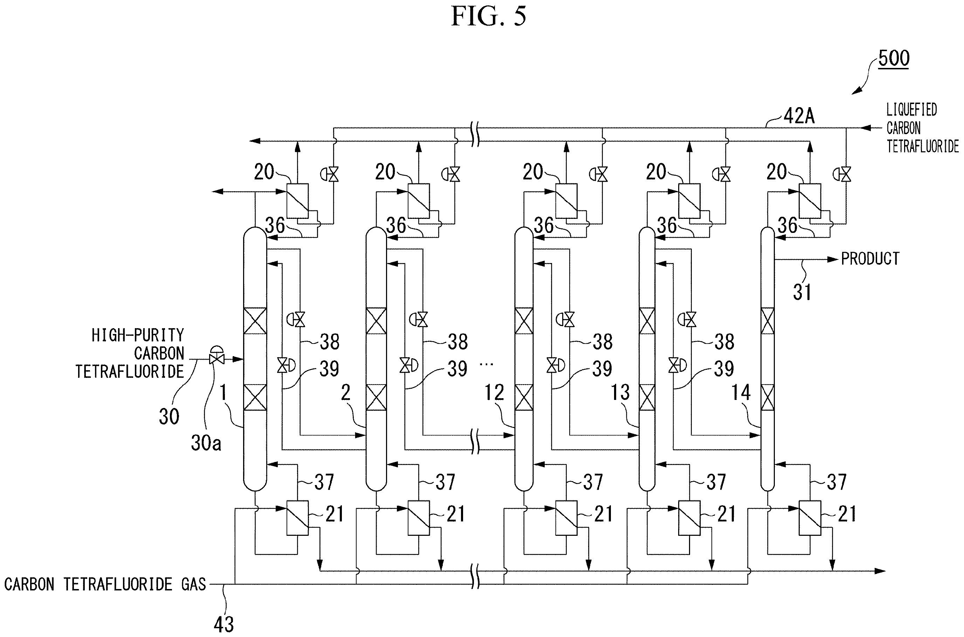

[FIG. 5] FIG. 5 is a diagram schematically illustrating a stable isotope enrichment device used in Comparative Example 2, and is a system diagram showing a main portion of the stable isotope enrichment device used for enriching carbon tetrafluoride containing a stable isotope in Comparative Example 2.

DETAILED DESCRIPTION OF THE PREFERRED EMBODIMENTS

Hereinafter, a stable isotope enrichment device and a stable isotope enrichment method, which are one embodiment of the present invention, will be described mainly with reference to FIG. 1 (see also FIG. 2 if necessary).

In addition, in the drawings used in the following explanation, in order to make the features easier to understand, the characteristic parts may be enlarged or simplified for convenience. Also, the materials and the like exemplified in the following description are merely examples, and the present invention is not limited thereto, and can be implemented with appropriate modifications within the scope of the invention.

As used in the present description, "isotope-depleted gas" and "isotope-depleted liquid" refers to a gas or liquefied gas discharged from a distillation column in which the concentration of molecules containing the target stable isotope is decreased below their natural abundance.

"Isotope-regenerated gas" and "isotope-regenerated liquid" means a gas and liquefied gas obtained by regenerating the "isotope-depleted gas" or "isotope-depleted liquid" above as a reusable raw material through an isotope exchange reaction.

<Stable isotope enrichment device>

A stable isotope enrichment device of the present embodiment will be described in detail below.

FIG. 1 is a system diagram showing a main portion of the stable isotope enrichment device 100 of the present embodiment.

The stable isotope enrichment device 100 of the present embodiment enriches a stable isotope by distillation. The stable isotope enrichment device 100 mainly includes a distillation column group in which a plurality of distillation columns are connected in a cascade, a raw material supply line 30, a product line 31, an isotope-depleted fluid withdraw line 32, an isotope exchange reactor 22, an isotope-regenerated fluid purifier 23, and an isotope regeneration fluid return line 33.

The illustrated stable isotope enrichment device 100 further includes a plurality of condensers 20, a plurality of reboilers 21, an isotope exchange reaction raw material supply line 34, and an isotope exchange reactor discharge line 35.

The stable isotope enrichment device 100 of the present embodiment is, for example, a device that enriches a stable isotope of hydrogen, carbon, oxygen or nitrogen by distillation.

Also, the stable isotope enrichment device 100 of the present embodiment is preferably used in the following cases (1) to (3), for example.

(1) a case in which the raw material is toxic or flammable, or there is a risk that the raw material will be discharged into the atmosphere and cause an environmental load
(2) a case in which there is a risk that substances converted by removal treatment of toxic substances used as raw materials will cause an environmental load when discharged into the atmosphere
(3) a case in which the raw material is a substance that leads to supply insecurity, such as a small amount of market supply or a scarcity value

Materials used in the stable isotope enrichment device 100 of the present embodiment are not particularly limited, and examples include $CO$, $CH_4$, $CF_4$, $CHF_3$, $NO$, $NH_3$, $NF_3$, $BF_3$, and the like.

The distillation column group includes, as described above, a plurality of distillation columns connected in a cascade.

In the following description, an n-th distillation column from the upstream end of the distillation column group is referred to as the n-th distillation column.

In the embodiment shown in FIG. 1, among a first distillation column 1 to the n-th distillation column n, only the first distillation column 1 to which the stable isotope molecule as a raw material is supplied and the n-th distillation column n are illustrated.

On the other hand, referring to the stable isotope enrichment device 200 shown in FIG. 2 used in the examples described later, the first distillation column 1 to the n-th distillation column n are connected in a cascade in order of column number. In the illustrated embodiment, the first distillation column 1 to a sixth distillation column 6 are connected in a cascade.

The first distillation column 1 to the n-th distillation column n distill cooled molecules containing stable isotopes at low or extremely low temperatures to enrich molecules containing a stable isotope with high vapor pressure on the top side, and enrich molecules containing a stable isotope with low vapor pressure on the bottom side.

When a plurality of distillation columns are connected in a cascade, "n" is a number of 2 or more.

On the other hand, for example, when a raw material containing a stable isotope with a purity close to that of the product is used and it can be enriched to the product concentration in one distillation column, n is 1 (i.e., one distillation column). In this case, the distillation column for being supplied with the raw material and the distillation column for recovering the product are the same.

In the distillation column group, the column diameter of the first distillation column 1 to which the raw material is supplied is usually the largest, and the column diameter gradually decreases toward the end. The distillation column group is designed in this manner because, in general, the first distillation column 1 on the upstream side has a large distillation load, and the n-th distillation column n on the downstream side has a smaller distillation load than the first distillation column 1.

In addition, each distillation column that constitutes the distillation column group may be any one of a packing column with regular packings, a packing column with random packings, and a tray column with rectification stages (trays).

One condenser 20 is provided for each distillation column (first distillation column 1 to n-th distillation column n). The condenser 20 is provided in a circulation line 36 of which ends are connected to different positions at the top of each distillation column. The condenser 20 has a function of liquefying gas that has arisen in the distillation column by exchanging heat and causing it to descend again in the distillation column.

Note that one condenser 20 may be provided for every several columns in the distillation columns (first distillation column 1 to n-th distillation column n).

One reboiler 21 is provided for each distillation column. The reboiler 21 is provided in a circulation line 37 of which ends are connected to different positions at the bottom of each distillation column. The reboiler 21 has a function of vaporizing liquid that has descended in the distillation column by exchanging heat and causing it to ascend again in the distillation column again.

Note that one reboiler 21 may be provided for every several columns in the distillation column (first distillation column 1 to n-th distillation column n).

One end of the raw material supply line 30 is connected to a middle portion of the first distillation column 1. The raw material supply line 30 is a passage for introducing a stable isotope as a raw material into a middle portion of the first distillation column (one distillation column) 1 of the distillation column group.

The middle portion of the distillation column means a position other than the top portion and the bottom portion of the distillation column.

In addition, the position (height) at which the raw material is introduced into the first distillation column 1 is not limited to the middle portion. The position at which the raw material is introduced into the first distillation column 1 is preferably a position at which the stable isotope concentration when the first distillation column 1 is in a steady state is close to the stable isotope concentration of the raw material.

Also, the raw material supply line 30 may be provided with a valve 30a as shown in FIG. 1.

It should be noted that one end of the raw material supply line 30 does not necessarily have to be connected to the first distillation column 1. When there is another distillation column on the primary side of the distillation column to which the raw material is supplied, one end of the raw material supply line 30 is connected to the second distillation column 2 and subsequent distillation columns.

It is preferable that the stable isotope used as a raw material have a high purity of 99.999% or more.

One end of the isotope-depleted fluid withdraw line 32 is connected to the first distillation column 1, and the other end is connected to the isotope exchange reactor 22 of which details will be described later. The connecting portion of the isotope-depleted fluid withdraw line 32 to the first distillation column 1 may be the top or the bottom depending on the molecules containing the target stable isotope. The isotope-depleted fluid withdraw line 32 draws an isotope-depleted gas or an isotope-depleted liquid in which the concentration of the molecules containing the target stable isotope is lower than the natural abundance from the first distillation column 1 of the distillation column group and introduces it into the isotope exchange reactor 22.

One end of the isotope-regenerated fluid return line 33 is connected to the isotope-regenerated fluid purifier 23 of which details will be described later, and the other end is connected to the middle portion of the first distillation column 1. The isotope-regenerated fluid return line 33 re-supplies an isotope-regenerated gas or an isotope-regenerated liquid regenerated in the isotope exchange reactor 22 and further purified in the isotope-regenerated fluid purifier 23 into the first distillation column 1.

The connecting portion of the isotope-regenerated fluid return line 33 at the first distillation column 1 is preferably above the raw material supply line 30, for example, when the vapor pressure of the molecules containing the target stable isotope is low. When the vapor pressure of the molecules containing the target stable isotopes is high, the isotope-regenerated fluid return line 33 is preferably connected below the raw material supply line 30. Note that the isotope-regenerated fluid return line 33 does not necessarily have to be connected to the same distillation column as the raw material supply line 30 is connected. When the raw material supply line 30 is connected to a distillation column after the second distillation column 2, the isotope-regenerated fluid return line 33 may be connected to the primary distillation column to which the raw material supply line 30 is connected.

One end of the isotope exchange reaction raw material supply line 34 is connected to the isotope exchange reactor 22. The isotope exchange reaction raw material supply line 34 is a line for supplying substances, which have the same stable isotope as the stable isotope of the isotope-depleted gas or the isotope-depleted liquid, are non-toxic, have no or extremely low environmental load, or have a lower environmental load compared to that of the isotope-depleted gas or the isotope-depleted liquid, into the isotope exchange reactor 22.

The isotope-depleted gas or the isotope-depleted liquid from first distillation column 1 is introduced into the isotope exchange reactor 22 through the isotope-depleted fluid withdraw line 32. In the isotope exchange reactor 22, the stable isotope in the molecules containing the stable isotope introduced from the first distillation column and other substances containing the same stable isotope as the stable isotope in the molecules introduced from the first distillation column 1. The isotope exchange reactor 22 regenerates the isotope-depleted gas or the isotope-depleted liquid so that the concentration of the molecules containing the stable isotope in the isotope-depleted gas or the isotope-depleted liquid approaches the natural abundance ratio by the isotope exchange reaction, and the isotope-regenerated gas or the isotope-regenerated liquid is produced.

Examples of the isotope exchange reaction in the isotope exchange reactor 22 include a catalyst heating reaction, a discharge reaction, an ion exchange method, and a photochemical reaction.

The inside of the isotope exchange reactor 22 can also be filled with substances (not shown) that cause an isotope exchange reaction with the isotope-depleted gas or the isotope-depleted liquid. When such a configuration is adopted, the isotope exchange reaction raw material supply line 34 may be omitted.

The isotope-regenerated fluid purifier 23 purifies the isotope-regenerated gas or the isotope-regenerated liquid produced by the isotope exchange reactor 22, and separates the isotope-regenerated gas or the isotope-regenerated liquid into stable isotope molecules as raw materials and other substances.

The method of purifying the isotope-regenerated gas or the isotope-regenerated liquid in the isotope-regenerated fluid purifier 23 is not particularly limited, but examples thereof include methods such as solidification purification, adsorption, membrane separation, and distillation.

A part of the vapor enriched in the molecules containing a stable isotope having a low vapor pressure at the bottom of each distillation column except for the final column is supplied to the top of the next distillation column through a passage 38.

The driving force for the vapor flow at this time is the pressure difference between the bottom of one distillation column and the top of the next distillation column. The vapor supplied to the top of the next distillation column is liquefied in the condenser 20 together with the ascending vapor in the distillation column and refluxed to the top of the distillation column.

In addition, a part of the reflux liquid enriched in the molecules containing a stable isotope having a high vapor pressure near the top of each distillation column except for the first distillation column 1 is supplied to the bottom of the previous distillation column through a passage 39.

The driving force for the flow of the reflux liquid at this time is the head pressure of the reflux liquid.

A compressor may be used as a driving force for the flow of the reflux liquid. When a compressor is used, it is also possible to compress the gas at the top of the distillation column and supply it into the previous distillation column. The reflux liquid supplied to the bottom of the previous distillation column is vaporized in the reboiler 21 together with the reflux liquid in the distillation column and refluxed to the bottom of the distillation column.

The product line 31 is a passage for withdrawing the component containing enriched molecules containing a stable isotope from the n-th distillation column n as a product. One end of the product line 31 is connected to a position closer to the bottom portion of the n-th distillation column (another distillation column) n located on the secondary side than the first distillation column (one distillation column) 1. The product withdrawn from the distillation column by the product line 31 is a highly enriched stable isotope component (for example, 99.9 atom % or more).

In addition, it is not always necessary to supply the raw material into the first distillation column 1. A distillation column, which serves as a recovery section, may be positioned upstream of the distillation column that supplies the raw material. In this case, one end of the isotope-depleted fluid withdraw line 32 is connected to the end of the distillation column which serves as a recovery section. Also, the distillation column from which the product is collected does not necessarily have to be the final column (n-th distillation column n), and may be a distillation column located in the middle of the distillation column group.

The effects obtained by the present invention will be described in more detail below.

In general, the isotope-depleted gas or the isotope-depleted liquid has a concentration of molecules containing the target stable isotope that is lower than the natural abundance ratio. Therefore, it cannot be reused as a raw material as it is. If the isotope-depleted gas or the isotope-depleted liquid can be regenerated so that the concentration of molecules containing the target stable isotope in the isotope-depleted gas or the isotope-depleted liquid approaches the natural abundance, "isotope-regenerated gas" or "isotope-regenerated liquid" produced can be reused as raw materials.

As a method for realizing the reuse of raw materials, there is a method using an isotope exchange reaction as employed in the present invention. The isotope exchange reaction is a reaction in which atoms (or atomic groups) in a molecule (or ion) are replaced with isotopes (or atomic groups containing an isotope) in other molecules (or ions).

Here, if the atom (or atomic group) to be replaced is denoted by X, the labeled atom (or atomic group) is given a * mark, and the molecule (or ion) involved in the exchange is denoted by A or B, the isotope exchange reaction generally has a relationship represented by the following formula (1). Intermediate substances (for example, $ABX_2$) may be present during the reaction from the left-hand side to the right-hand side.

$$AX + BX^* \; \rightarrow \; AX^* + BX \quad (1)$$

When, in the formula (1) above, AX is equivalent to the isotope-depleted gas or the isotope-depleted liquid in which $X^*$ has a concentration lower than the natural abundance ratio, and $BX^*$ is a substance containing $X^*$ in the natural abundance ratio, the concentration of $X^*$ in $AX^*$ after the isotope exchange reaction approaches the natural abundance ratio. Furthermore, when the mixing ratio of $BX^*$ to AX is sufficiently large, the concentration of $X^*$ in $AX^*$ is approximately the same as the natural abundance ratio.

The isotope-regenerated gas or the isotope-regenerated liquid in which the concentration of molecules containing the stable isotope has been improved close to the natural abundance ratio by the isotope exchange reaction represented by the above formula (1) can be supplied again into the distillation column as a raw material. In this case, the substance discharged out of the system corresponds to BX. Therefore, the substance corresponding to $BX^*$ used in the isotope exchange reaction can be a substance which is non-toxic and has no or an extremely low environmental load, or substance which has a smaller environmental load than that of AX. It is possible to decrease the load on the environment by the isotope-depleted gas or the isotope-depleted liquid.

In an enrichment device for obtaining $^{15}$N stable isotope by distillation of nitric oxide, for example, it is conceivable to carry out an isotope exchange reaction between NO gas discharged from the distillation column, in which the concentration of $^{15}$N is lower than the natural abundance ratio, and general $N_2$ gas. In this case, the NO gas having a concentration of $^{15}$N approximately equal to the natural abundance is re-supplied into the distillation column. Also, in this case, a large amount of $N_2$ gas is discharged, but since $N_2$ is a substance that exists in the air, the load on the environment is small.

That is, in the present invention, the isotope-depleted gas or the isotope-depleted liquid is subjected to the isotope exchange reaction with other substances having the same stable isotope as the stable isotope in the isotope-depleted gas or the isotope-depleted liquid, and the isotope-depleted gas or the isotope-depleted liquid is regenerated such that the concentration of the stable isotope in the isotope-depleted gas or the isotope-depleted liquid approaches the natural abundance ratio, and then the isotope-regenerated gas or the isotope-regenerated liquid obtained is re-supplied into the distillation section as a raw material. This makes it possible to decrease the discharge amount of the isotope-depleted gas or the isotope-depleted liquid as a raw material, which is toxic, combustible, or has an environmental load when discharged into the atmosphere.

In addition, the present invention is very useful for the purpose of enriching stable isotopes using substances of which the supply amount is unstable due to circumstances such as low market volume and high scarcity value. In the present invention, almost all of the raw material is the raw material which is regenerated to make the concentration of the molecules containing the target stable isotope closer to the natural abundance ratio, and re-supplied. For this reason, the amount of raw material which is necessary to be newly supplied is almost the same as the amount withdrawn as a product. Therefore, even considering the loss due to by-products generated by the isotope exchange reaction, the total amount of the raw materials used can be greatly decreased.

<Stable isotope enrichment method>

Next, the stable isotope enrichment method (hereinafter sometimes simply referred to as the "enrichment method") of the present embodiment will be explained.

In addition, in the present embodiment, a stable isotope enrichment method using the stable isotope enrichment device 100 described above will be described with reference to FIG. 1.

The enrichment method of the present embodiment is a method for enriching a stable isotope by distillation. That is, the enrichment method of the present embodiment is a method in which the isotope-depleted gas or the isotope-depleted liquid, of which the concentration of the molecules containing the target stable isotope is lower than the natural abundance ratio and is withdrawn from the first distillation column (one distillation column) 1 of the distillation column group in which a plurality of distillation columns are connected in a cascade, is subjected to the isotope exchange reaction with other substances having the same stable isotope as that in the isotope-depleted gas or the isotope-depleted liquid using the stable isotope enrichment device 100.

The enrichment method of the present embodiment includes a re-supplying step in which the isotope-depleted gas or the isotope-depleted liquid is regenerated such that the concentration of the stable isotope in the isotope-depleted gas or the isotope-depleted liquid approaches the natural abundance ratio to produce the isotope-regenerated gas or the isotope-regenerated liquid, and the isotope-regenerated gas or the isotope-regenerated liquid obtained is re-supplied into the first distillation section 1.

Specifically, first, the raw material in gaseous or liquid state is supplied into the first distillation column 1 through the raw material supply line 30. At this time, for example, a cold heat source is supplied to the condenser 20 to cool and liquefy the raw material containing the stable isotope, and the liquefied raw material is accumulated at the bottom of each distillation column.

A heating source is supplied into the reboiler 21 after the amount of liquefied raw material required for the operation of the reboiler 21 has accumulated at the bottom of each distillation column.

After confirming that each distillation column is in a stable state, continuous introduction of the raw material (stable isotope) from the raw material supply line 30 into the first distillation column 1 is started, and the enrichment operation of the stable isotope enrichment device 100 is started.

That is, after the start-up of the stable isotope enrichment device 100 is completed, the product is withdrawn from the n-th distillation column n through the product line 31.

Note that, as described above, in an enrichment device for distilling the stable isotope, when the isotope-depleted gas or the isotope-depleted liquid of which the concentration of molecules containing the target heavy isotope is decreased is discharged outside the system, and the isotope-depleted gas or the isotope-depleted liquid is a combustible and toxic gas or a substance that causes an environmental load when released into the atmosphere, it may have an adverse effect on the environment.

On the other hand, the stable isotope enrichment method using the stable isotope enrichment device 100 includes the re-supplying step in which the isotope-depleted gas or the isotope-depleted liquid withdrawn from the first distillation column 1 is subjected to the isotope exchange reaction with other substances having the same stable isotope as the stable isotope in the isotope-depleted gas or the isotope-depleted liquid, and the isotope-depleted gas or the isotope-depleted liquid withdrawn from the first distillation column 1 is regenerated such that the concentration of the stable isotope in the isotope-depleted gas or the isotope-depleted liquid approaches the natural abundance ratio, and the isotope-regenerated gas or the isotope-regenerated liquid obtained is re-supplied into the first distillation column 1.

As a result, it is possible to decrease the amount of the raw materials used while reducing the environmental load in the stable isotope enrichment process. This makes it possible to obtain high-purity molecules containing the target stable isotope at the desired concentration at low cost while maintaining a favorable process environment.

Furthermore, according to the enrichment method of the present embodiment, it is possible to reuse "the isotope-regenerated gas" or "the isotope-regenerated liquid" as a raw material by regenerating the isotope-depleted gas or the isotope-depleted liquid so that the concentration of molecules containing the target stable isotope in the isotope-depleted gas or the isotope-depleted liquid approaches the natural abundance ratio.

In addition, in the enrichment method of the present embodiment, any one of catalyst heating reaction, discharge reaction, ion exchange reaction, and photochemical reaction can be adopted as the isotope exchange reaction.

<Other embodiments>

Although examples of the stable isotope enrichment device and the stable isotope enrichment method according to the present invention have been described above with reference to the embodiments, the present invention is not limited to the embodiments above. Each configuration and combination thereof in the embodiment above are examples, and addition, omission, replacement, and other modifications of the configuration are possible without departing from the scope of the present invention.

For example, the condenser 20 and the reboiler 21 are not essential components for each distillation column. The condenser 20 and the reboiler 21 may be omitted if the gas rises sufficiently due to the pressure difference between the front and rear distillation columns, a pump, a blower, or the like. Also, the connection positions of the passage 38 and the passage 39 to the distillation column may be reversed. The passage 38 and the passage 39 may be provided such that a part of the reflux liquid near the top of the distillation column is supplied to the bottom of the next distillation column via the passage 38, or a part of the vapor at the bottom of the distillation column is supplied to the top of the previous column via the passage 39.

<Effects>

As described above, the stable isotope enrichment device 100 of the present embodiment includes the isotope exchange reactor 22 in which the isotope-depleted gas or the isotope-depleted liquid withdrawn from the first distillation column 1, in which the concentration of the molecules containing the target stable isotope is lower than the natural abundance ratio, is subjected to the isotope exchange reaction with other substances having the same stable isotope as the stable isotope in the isotope-depleted gas or the isotope-depleted liquid, the isotope-depleted gas or the isotope-depleted liquid is regenerated such that the concentration of the molecules containing the target stable isotope in the isotope-depleted gas or the isotope-depleted liquid approaches the natural abundance ratio, and the isotope-regenerated gas or the isotope-regenerated liquid is produced.

Since the stable isotope enrichment device 100 of the present embodiment includes the isotope exchange reactor 22, the discharge amount of the isotope-depleted gas or the isotope-depleted liquid containing toxic or combustible substances or substances that cause environmental load in the atmosphere can be decreased, and it becomes possible to reuse these substances as the raw material.

Therefore, it is possible to decrease the amount of raw materials used while reducing the environmental load in the stable isotope enrichment process. Therefore, it becomes possible to efficiently obtain substances containing the target stable isotope with high purity at low cost while maintaining a favorable process environment.

In addition, the stable isotope enrichment method of the present embodiment includes the re-supplying step in which the isotope-depleted gas or the isotope-depleted liquid of which the concentration of the molecules containing the target stable isotope is lower than the natural abundance ratio and which is withdrawn from the first distillation column is subjected to the isotope exchange reaction with other substances having the same stable isotope such that the concentration of the molecules containing a stable isotope in the isotope-depleted gas or the isotope-depleted liquid approaches the natural abundance ratio, the isotope-depleted gas or the isotope-depleted liquid is regenerated to produce the isotope-regenerated gas or the isotope-regenerated liquid, and the isotope-regenerated gas or the isotope-regenerated liquid is re-supplied into the first distillation column.

Since the stable isotope enrichment method according to the present invention includes the step in which the isotope exchange reaction is carried out, it is possible to decrease the amount of the raw material used while reducing the environmental load in the process of enriching stable isotopes, as described above. Therefore, it is possible to efficiently obtain substances containing the target stable isotope with high purity at low cost while maintaining a favorable process environment.

EXAMPLES

Hereinafter, the stable isotope enrichment device and the stable isotope enrichment method of the present invention will be described in more detail with reference to examples. However, the present invention is not limited to the following examples, and can be modified as appropriate without changing the gist of the invention.

<Example 1>

In Example 1, $^{15}N^{18}O$ was enriched using a nitric monoxide stable isotope enrichment device 200 having the configuration according to the present invention shown in FIG. 2.

The stable isotope enrichment device 200 used in Example 1 basically has the same configuration as the stable isotope enrichment device 100 shown in FIG. 1, but differs in that the distillation column group includes six distillation columns. The first distillation column 1 to a sixth distillation column 6 are distillation columns filled with regular packings.

Table 1 below shows the isotopic composition of the raw material nitric monoxide supplied into the first distillation column 1.

In addition, the isotope exchange reactor 22 was supplied with a gas in which oxygen and nitrogen were mixed at a volume ratio of 1:1. Tables 2 and 3 below show the isotopic compositions of the oxygen and nitrogen supplied.

Further, in Example 1, the $^{15}N^{18}O$ product was withdrawn from the bottom of the sixth distillation column 6, and some by-products were withdrawn from the bottom of the third distillation column 3.

The flow rates shown in (1) to (6) below are shown in Table 4 below.

(1) Flow rate of raw material nitrogen monoxide
(2) Flow rate of $^{15}N^{18}O$ product nitrogen monoxide withdrawn
(3) Flow rate of nitric monoxide supplied from the first distillation column 1 into the isotope exchange reactor 22
(4) Flow rate of mixed gas of oxygen and nitrogen supplied from the isotope exchange reaction raw material supply line 34
(5) Flow rate of nitric monoxide returned from the isotope-regenerated fluid purifier 23 into the middle portion of the first distillation column 1
(6) Flow rate of by-products

The isotope exchange reactor 22 utilizes a photochemical reaction caused by ultraviolet light irradiation.

The isotope composition of the nitric monoxide before and after the isotope exchange reactor 22 in the flow direction is shown in Table 5 below.

In the isotope-regenerated fluid purifier 23, the adsorption method and the distillation method were used to separate nitrogen monoxide from other substances.

The nitrogen monoxide stable isotope enrichment device 200 is equipped with the isotope scrambler 24. The isotope scrambler 24 is a device that generates isotope scrambling, which is an exchange reaction that randomly rearranges atoms.

Also, in the stable isotope enrichment device 200, one end of an isotope-enriched gas withdraw line 40 is connected to the bottom portion of the fourth distillation column 4 and the other end thereof is connected to the isotope scrambler 24. A part or all of the nitrogen monoxide was withdrawn from the fourth distillation column 4 and supplied into the isotope scrambler 24.

Nitrogen monoxide that has been subjected to the isotope exchange reaction in the isotope scrambler 24 was returned to the top portion of the fifth distillation column 5 through the isotope-enriched gas return line 41.

Liquefied methane was used as the cold source of the condenser 20, and the liquefied methane was supplied into each condenser through the liquefied methane supply line 42.

An electric heater was used as the heat source for the reboiler 21.

Tables 6 and 7 below show the abundance ratio of the molecules and the stable isotopes in the product withdrawn from the sixth distillation column 6, which is the final stage, when the entire enrichment device is stable.

Also, the total amount of heat exchange required by the condenser 20 and the reboiler 21 was 160 kW.

[Table 1]

| Molecule containing stable isotope | Molecular weight | Abundance ratio [%] |
|---|---|---|
| $^{14}N^{16}O$ | 30 | 99.4 |
| $^{14}N^{17}O$ | 31 | 0.0 |
| $^{14}N^{18}O$ | 32 | 0.2 |
| $^{15}N^{16}O$ | 31 | 0.4 |
| $^{15}N^{17}O$ | 32 | 0.0 |
| $^{15}N^{18}O$ | 33 | 0.0 |

[Table 2]

| Molecule containing stable isotope | Molecular weight | Abundance ratio [%] |
|---|---|---|
| $^{16}O_2$ | 32 | 99.5 |
| $^{16}O^{17}O$ | 33 | 0.1 |
| $^{16}O^{18}O$ | 34 | 0.4 |
| 17 02 | 34 | 0.0 |
| $^{17}O^{18}O$ | 35 | 0.0 |
| $^{18}O_2$ | 36 | 0.0 |

[Table 3]

| Molecule containing stable isotope | Molecular weight | Abundance ratio [%] |
|---|---|---|
| $^{14}N_2$ | 28 | 99.3 |
| $^{14}N^{15}N$ | 29 | 0.7 |
| $^{15}N_2$ | 30 | 0.0 |

[Table 4]

| Number | Flow rate |
|---|---|
| (1) | 2 $Nm^3/hr$ |
| (2) | 7 NL/hr |
| (3) | 20 $Nm^3/hr$ |
| (4) | 2,000 $Nm^3/hr$ |
| (5) | 18 $Nm^3/hr$ |
| (6) | 9 NL/hr |

[Table 5]

| Molecule containing stable isotope | Molecular weight | Before reaction [%] | After reaction [%] |
|---|---|---|---|
| $^{14}N^{16}O$ | 30 | 99.5 | 99.4 |
| $^{14}N^{17}O$ | 31 | 0.0 | 0.0 |
| $^{14}N^{18}O$ | 32 | 0.2 | 0.2 |
| $^{15}N^{16}O$ | 31 | 0.3 | 0.4 |
| $^{15}N^{17}O$ | 32 | 0.0 | 0.0 |
| $^{15}N^{18}O$ | 33 | 0.0 | 0.0 |

[Table 6]

| Molecule containing stable isotope | Molecular weight | Abundance ratio [%] |
|---|---|---|
| $^{14}N^{16}O$ | 30 | 0.0 |
| $^{14}N^{17}O$ | 31 | 0.0 |
| $^{14}N^{18}O$ | 32 | 0.7 |
| $^{15}N^{16}O$ | 31 | 0.0 |
| $^{15}N^{17}O$ | 32 | 0.0 |
| $^{15}N^{18}O$ | 33 | 99.3 |

[Table 7]

| Stable isotope | Atomic weight | Abundance ratio [%] |
|---|---|---|
| $^{15}N$ | 15 | 99.3 |
| $^{18}O$ | 18 | 100.0 |

<Comparative Example 1>

In Comparative Example 1, $^{15}N^{18}O$ was enriched using a nitrogen monoxide stable isotope enrichment device 300 shown in FIG. 3. The stable isotope enrichment device 300 shown in FIG. 3 differs from the stable isotope enrichment device 200 according to the present invention shown in FIG. 2 in that it does not include the isotope exchange reactor 22, the isotope-regenerated fluid purifier 23, and the lines before and after them.

Further, the stable isotope enrichment device 300 includes the distillation column group including 6 distillation columns similarly to the stable isotope enrichment device 200. The first to sixth distillation columns 1 to 6 are distillation columns filled with regular packings.

The isotope composition of the raw material nitrogen monoxide supplied into the first distillation column 1 was as shown in Table 1 above.

Further, in Comparative Example 1, the $^{15}N^{18}O$ product was withdrawn from the bottom of the sixth distillation column 6, and some by-products were withdrawn from the bottom of the third distillation column 3.

The flow rates shown in (1) to (3) below are shown in Table 8 below.

(1) Flow rate of raw material nitrogen monoxide
(2) Flow rate of $^{15}N^{18}O$ product nitrogen monoxide withdrawn
(3) Flow rate of by-products

The stable isotope enrichment device 300 shown in FIG. 3 includes the isotope scrambler 24 similar to the stable isotope enrichment device 200 shown in FIG. 2.

Also in the stable isotope enrichment device 300, one end of the isotope enriched gas drawing line 40 is connected to the bottom portion of the fourth distillation column 4 and the other end thereof is connected to the isotope scrambler 24. A part or all of the nitrogen monoxide was withdrawn from the fourth distillation column 4 and supplied into the isotope scrambler 24.

Nitrogen monoxide that has been subjected to the isotope exchange reaction in the isotope scrambler 24 was returned to the top portion of the fifth distillation column 5 through the isotope-enriched gas return line 41.

Liquefied methane was used as the cold source of the condenser 20, and the liquefied methane was supplied to each condenser through the liquefied methane supply line 42.

An electric heater was used as the heat source for the reboiler 21.

Tables 9 and 10 below show the abundance ratio of the molecules and the stable isotopes in the product withdrawn from the sixth distillation column 6, which is the final stage, when the entire enrichment device is stable.

Also, the total amount of heat exchange required by the condenser 20 and the reboiler 21 was 160 kW.

[Table 8]

| Number | Flow rate |
|--------|-----------|
| (1) | 20 Nm$^3$/hr |
| (2) | 8 NL/hr |
| (3) | 9 NL/hr |

[Table 9]

| Molecule containing stable isotope | Molecular weight | Abundance ratio [%] |
|-----------------------------------|------------------|---------------------|
| $^{14}N^{16}O$ | 30 | 0.0 |
| $^{14}N^{17}O$ | 31 | 0.0 |
| $^{14}N^{18}O$ | 32 | 0.7 |
| $^{15}N^{16}O$ | 31 | 0.0 |
| $^{15}N^{17}O$ | 32 | 0.0 |
| $^{15}N^{18}O$ | 33 | 99.3 |

[Table 10]

| Stable isotope | Atomic weight | Abundance ratio [%] |
|---|---|---|
| $^{15}N$ | 15 | 99.3 |
| $^{18}O$ | 18 | 100.0 |

In Comparative Example 1, the amount of the raw material amount was 20 $Nm^3$/hr and the total amount of the product was 17 NL/hr. Therefore, it was necessary to remove nitrogen monoxide at a flow rate substantially equal to that of the raw material discharged from the top of the first column. In the ammonia catalytic reduction method, it is necessary to add more than the same amount of ammonia as that of nitrogen monoxide, which increases the amount of toxic gas handled. For this reason, the manufacturing cost increased due to the implementation of safety measures and the like.

On the other hand, in Example 1, the additive gas was oxygen and nitrogen, and most of the discharge gas was also oxygen and nitrogen separated by the isotope-regenerated fluid purifier 23. Therefore, it was possible to manufacture at low cost without the need for removal treatment. In addition, the amount of nitrogen monoxide newly required as the raw material was also decreased to 1/10.

In addition, the required heat exchange amount, product concentration, and product amount were approximately the same in each of Example 1 and Comparative Example 1.

<Example 2>

In Example 2, $^{13}CF_4$ was enriched using a carbon tetrafluoride stable isotope enrichment device 400 having the configuration according to the present invention as shown in FIG. 4. The stable isotope enrichment device 400 used in Example 2 differs from the stable isotope enrichment device 100 in that the distillation column group includes 14 distillation columns. The first distillation column 1 to the fourteenth distillation column 14 are distillation columns filled with regular packings.

The isotope composition of the raw material carbon tetrafluoride supplied into the first distillation column 1 was as shown in Table 11 below.

The isotope exchange reactor 22 was filled with a palladium-supported carbon catalyst.

As the metal supported on the carbon carrier, in addition to palladium, for example, zinc, nickel, cesium, cobalt, vanadium, calcium, manganese, magnesium, tungsten, titanium, platinum, or copper can also be used.

In addition to the carbon described above, for example, aluminum phosphate, alumina, a composite of alumina and zirconium oxide, and the like can also be used as the catalyst carrier.

The amount of the catalyst filled in the isotope exchange reactor 22 was about 30 kg, and the isotope composition of carbon in the carbon support was as shown in Table 12 below.

Further, in Example 2, the $^{13}CF_4$ product was withdrawn from the bottom of the fourteenth distillation column 14.

The flow rates shown in (1) to (4) below are shown in Table 13 below.

(1) Flow rate of raw material carbon tetrafluoride
(2) Flow rate of $^{13}CF_4$ product carbon tetrafluoride withdrawn
(3) Flow rate of carbon tetrafluoride supplied from the first distillation column 1 into the isotope exchange reactor 22
(4) Flow rate of carbon tetrafluoride returned from the isotope-regenerated fluid purifier 23 to the middle portion of the first distillation column 1

The isotope exchange reactor 22 utilizes catalytic heating reaction.

The isotope composition of carbon tetrafluoride before and after the isotope exchange reactor 22 in the flow direction was as shown in Table 14 below.

Also, in the isotope-regenerated fluid purifier 23, the distillation method was used to separate carbon tetrafluoride from other substances.

Liquid carbon tetrafluoride was used as the cold source of the condenser 20, and the liquefied carbon tetrafluoride was supplied into each condenser through the liquefied carbon tetrafluoride supply line 42A.

Carbon tetrafluoride gas was used as the heat source of the reboiler 21, and carbon tetrafluoride gas was supplied into each reboiler 21 through a carbon tetrafluoride supply gas line 43.

Tables 15 and 16 below show the abundance ratio of the molecules and the stable isotopes in the product withdrawn from the sixth distillation column 14, which is the final stage, when the entire enrichment device is stable

Also, the total amount of heat exchange required by the condenser 20 and the reboiler 21 was 1180 kW.

[Table 11]

| Molecule containing stable isotope | Molecular weight | Abundance ratio [%] |
|---|---|---|
| $^{12}CF_4$ | 88 | 98.9 |
| $^{13}CF_4$ | 89 | 1.1 |

[Table 12]

| Stable isotope | Atomic weight | Abundance ratio [%] |
|---|---|---|
| $^{12}C$ | 12 | 98.9 |
| $^{13}C$ | 13 | 1.1 |

[Table 13]

| Number | Flow rate |
|---|---|
| (1) | 1 $Nm^3$/hr |
| (2) | 59 NL/hr |
| (3) | 20 $Nm^3$/hr |
| (4) | 19 $Nm^3$/hr |

[Table 14]

| Molecule containing stable isotope | Molecular weight | Before reaction [%] | After reaction [%] |
|---|---|---|---|
| $^{12}CF_4$ | 88 | 99.3 | 99.0 |
| $^{13}CF_4$ | 89 | 0.7 | 1.0 |

[Table 15]

| Molecule containing stable isotope | Molecular weight | Abundance ratio [%] |
|---|---|---|
| $^{12}CF_4$ | 88 | 0.5 |
| $^{13}CF_4$ | 89 | 99.5 |

[Table 16]

| Stable isotope | Atomic weight | Abundance ratio [%] |
|---|---|---|
| $^{13}C$ | 13 | 99.5 |

<Comparative Example 2>

In Comparative Example 2, the carbon tetrafluoride stable isotope enrichment device 500 shown in FIG. 5 was used to enrich $^{13}CF_4$.

The stable isotope enrichment device 500 shown in FIG. 5 differs from the stable isotope enrichment device 400 according to the present invention shown in FIG. 4 in that it does not include the isotope exchange reactor 22, the isotope-regenerated fluid purifier 23, and the lines before and after them. In the stable isotope enrichment device 500, the distillation column group includes 14 distillation columns, similarly to the stable isotope enrichment device 400, and the first distillation column 1 to the fourteenth distillation columns 14 are filled with regular packings.

The isotope composition of the raw material carbon tetrafluoride supplied into the first distillation column 1 was as shown in Table 11 above.

Also in Comparative Example 2, the $^{13}CF_4$ product was withdrawn from the bottom of the fourteenth distillation column 14.

The flow rates shown in (1) and (2) below are shown in Table 17 below.

(1) Flow rate of raw material carbon tetrafluoride
(2) Flow rate of $^{13}CF_4$ product carbon tetrafluoride withdrawn

Liquid carbon tetrafluoride was used as a cold source for the condenser 20, and the liquid carbon tetrafluoride was supplied into each condenser through the liquefied carbon tetrafluoride supply line 42A.

Carbon tetrafluoride gas was used as the heat source of the reboiler 21, and carbon tetrafluoride gas was supplied into each reboiler 21 through the carbon tetrafluoride gas supply line 43.

Tables 18 and 19 below show the abundance ratio of the molecules and the stable isotopes in the product withdrawn from the sixth distillation column 14, which is the final stage, when the entire enrichment device is stable

Also, the total amount of heat exchange required by the condenser 20 and the reboiler 21 was 1170 kW.

[Table 17]

| Number | Flow rate |
|--------|-----------|
| (1) | 20 Nm$^3$/hr |
| (2) | 59 NL/hr |

[Table 18]

| Molecule containing stable isotope | Molecular weight | Abundance ratio [%] |
|-----------------------------------|------------------|---------------------|
| $^{12}CF_4$ | 88 | 0.4 |
| $^{13}CF_4$ | 89 | 99.6 |

[Table 19]

| Stable isotope | Atomic weight | Abundance ratio [%] |
|----------------|---------------|---------------------|
| $^{13}C$ | 13 | 99.6 |

Comparative Example 2 required the raw material carbon tetrafluoride at a flow rate of 20 Nm$^3$/hr. In this case, approximately 690 tons of raw material is required per year in terms of mass. According to "Gas Diorama 2020" (Gas Review Co., Ltd., 2020, P88), the annual domestic sales volume of carbon tetrafluoride in Japan is about 650 tons, accounting for about 25% of the global demand. However, in reality, it is difficult to use about 27% of the world demand for raw materials in one plant, and the scale of the plant is limited.

On the other hand, in Example 2, carbon tetrafluoride required as a raw material is about 38 tons per year. It was possible to greatly decrease the amount of raw materials used, and this amount of raw materials could be supplied sufficiently.

Furthermore, in Comparative Example 2, about 680 tons of carbon tetrafluoride is discharged into the atmosphere annually, which is equivalent to about 5 million tons of $CO_2$ discharge, and has a very large load on the environment. For this reason, in the case of Comparative Example 2, it is necessary to convert carbon tetrafluoride into substances which have less load on the environment before discharging, and the cost for this is added.

On the other hand, in Example 2, although some discharges such as $CO_2$ generated as by-products in the isotope exchange reactor 22 are observed, the discharge amount is very small compared to the $CO_2$ conversion in Comparative Example 2.

The results above can be obtained not only for molecules containing the stable isotope in Examples 1 and 2, but also when the present invention is applied to other molecules containing the stable isotope. It is possible to decrease the amount of toxic or combustible substances, or discharged gases or discharged liquids that cause an environmental load when discharged into the atmosphere by applying the present invention to the enrichment of molecules containing a stable isotope such as CO, $CH_4$, $CF_4$, $CHF_3$, $NH_3$, $NF_3$, and $BF_3$. In addition, when a substance of which supply is unstable is used as a raw material, it is possible to decrease the amount used.

INDUSTRIAL APPLICABILITY

According to the stable isotope enrichment device and the stable isotope enrichment method of the present invention, it is possible to decrease the amount of substances that are toxic or combustible, or substances that cause an environmental load in the atmosphere, and decrease the amount of the raw material used.

Therefore, the present invention is extremely useful for providing stable isotopes used in drug discovery and organic synthesis, stable isotopes used in infrared and Raman spectroscopy using differences in spectroscopic properties in addition to stable isotopes used as tracers in the fields of natural science and medicine.

EXPLANATION OF REFERENCE NUMERALS

| 100, 200, | 400 stable isotope enrichment device |
|---|---|
| 1, 2, 3, 4, 5, 6 to 14, (n) | distillation column (first distillation column 1 to n-th distillation column n; distillation column group) |
| 20 | condenser |
| 21 | reboiler |
| 22 | isotope exchange reactor |
| 23 | isotope-regenerated fluid purifier |
| 24 | isotope scrambler |
| 30 | raw material supply line |
| 30a | valve |
| 31 | product line |
| 32 | isotope-depleted fluid withdraw line |
| 33 | isotope-regenerated fluid return line |
| 34 | isotope exchange reaction raw material supply line |
| 35 | isotope exchange reactor drain line |
| 36, 37 | circulation line |
| 38, 39 | passage |
| 40 | isotope-enriched gas withdraw line |
| 41 | isotope-enriched gas return line |
| 42 | liquefied methane supply line |
| 42A | liquefied carbon tetrafluoride supply line |
| 43 | carbon tetrafluoride gas supply line |

**Claims**

1. A stable isotope enrichment device for enriching a stable isotope by distillation, comprising:

a distillation column group in which a plurality of distillation columns are connected in a cascade;
a raw material supply line that supplies a raw material into one of the distillation columns in the distillation column groups;
a product line that withdraws a product from another distillation column located on the secondary side of the one distillation column in the distillation column group;
an isotope-depleted fluid withdraw line that withdraws an isotope-depleted gas or an isotope-depleted liquid of which a concentration of molecules containing the target stable isotope is lower than a natural abundance ratio, and which is from the one distillation column or another distillation column located on the primary side of the one distillation column in the distillation column group;
an isotope exchange reactor at which the isotope-depleted gas or the isotope-depleted liquid introduced from the isotope-depleted fluid withdraw line is subjected to an isotope exchange reaction with other substances including the same stable isotope as the target stable isotope, the isotope-depleted gas or the isotope-depleted liquid is regenerated such that the concentration of the molecules containing the target stable isotope in the isotope-depleted gas or the isotope-depleted liquid approaches a natural abundance ratio, and an isotope-regenerated gas or an isotope-regenerated liquid is produced; and

an isotope-regenerated fluid return line that re-supplies the isotope-regenerated gas or the isotope-regenerated liquid produced in the isotope exchange reactor into the one distillation column.

2. The stable isotope enrichment device according to Claim 1, wherein the isotope exchange reaction in the isotope exchange reactor is any of a catalyst heating reaction, a discharge reaction, an ion exchange method, and a photochemical reaction.

3. An isotope enrichment method for enriching a stable isotope by distillation, including:
a re-supplying step in which an isotope-depleted gas or an isotope-depleted liquid, of which a concentration of molecules containing the target stable isotope is lower than a natural abundance ratio and which is withdrawn from one distillation column or another distillation column located on the primary side of the one distillation column in a distillation column group in which a plurality of distillation columns are connected in a cascade, is subjected to an isotope exchange reaction with other substances containing the same stable isotope as the target stable isotope, the concentration of molecules containing the target stable isotope in the isotope-depleted gas or the isotope-depleted liquid approaches the natural abundance ratio, the isotope-depleted gas or the isotope-depleted liquid is regenerated, and an isotope-regenerated gas or an isotope-regenerated liquid is produced, and the isotope-regenerated gas or the isotope-regenerated liquid is re-supplied into the one distillation column.

4. The stable isotope enrichment method according to Claim 3, wherein the isotope exchange reaction in the isotope exchange reactor is any of a catalyst heating reaction, a discharge reaction, an ion exchange method, and a photochemical reaction.

# FIG. 1

FIG. 2

EP 4 268 942 A1

# FIG. 3

EP 4 268 942 A1

FIG. 4

EP 4 268 942 A1

# FIG. 5

EP 4 268 942 A1

## INTERNATIONAL SEARCH REPORT

| International application No. |
| --- |
| **PCT/JP2021/045097** |

**A.    CLASSIFICATION OF SUBJECT MATTER**

*B01D 59/04*(2006.01)i; *C01B 21/24*(2006.01)i; *C07C 17/383*(2006.01)i; *C07C 19/08*(2006.01)i
FI:    B01D59/04; C01B21/24 Z; C07C17/383; C07C19/08

According to International Patent Classification (IPC) or to both national classification and IPC

**B.    FIELDS SEARCHED**

Minimum documentation searched (classification system followed by classification symbols)

B01D59/04, B01D59/28-59/33, B01D3/00-3/42, F25J3/00-3/08, C01B4/00, C01B21/24, C07C17/383, C07C19/08

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2019-513556 A (AKCIONERNOE OBSHHESTVO RADIEVYY INST IMENI V G KHLOPINA) 30 May 2019 (2019-05-30)<br>claims 1-2, paragraphs [0017]-[0035], fig. 2 | 1-4 |
| A | JP 2018-164884 A (TAIYO NIPPON SANSO CORP) 25 October 2018 (2018-10-25) | 1-4 |
| A | WO 2000/027509 A1 (NIPPON SANSO CORPORATION) 18 May 2000 (2000-05-18) | 1-4 |
| A | CN 201493052 U (SHANGHAI RESEARCH INSTITUTE OF CHEMICAL INDUSTRY) 02 June 2010 (2010-06-02) | 1-4 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **26 January 2022** | **08 February 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/045097**

| Patent document cited in search report | | | Publication date (day/month/year) | Patent family member(s) | | | Publication date (day/month/year) |
|---|---|---|---|---|---|---|---|
| JP | 2019-513556 | A | 30 May 2019 | WO | 2017/176157 | A1 | |
| | | | | RU | 2016113333 | A | |
| | | | | RU | 2632697 | C1 | |
| | | | | JP | 6902091 | B2 | |
| JP | 2018-164884 | A | 25 October 2018 | JP | 6606522 | B2 | |
| WO | 2000/027509 | A1 | 18 May 2000 | US | 6461583 | B1 | |
| | | | | JP | 4467190 | B2 | |
| CN | 201493052 | U | 02 June 2010 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4327287 B
- JP 2018164884 A

- JP 4309162 B

**Non-patent literature cited in the description**

- Separation of Nitrogen and Oxygen Isotopes by Nitric Oxide Low Temperature Distillation. **SHOHEI ISOMURA ; YASUHIKO TONOOKA ; HAYATO KA-ETSU.** RADIO ISOTOPES. Japan Radioisotope Association, 15 February 1987, vol. 36, 57-63